(19)

Europäisches
Patentamt

European
Patent Office

Office européen
des brevets

(11) **EP 3 893 247 A1**

(12) **EUROPEAN PATENT APPLICATION**

(43) Date of publication:
**13.10.2021 Bulletin 2021/41**

(51) Int Cl.:
*G16H 50/20* (2018.01)      *A61B 5/08* (2006.01)
*A61B 5/11* (2006.01)      *A61B 5/113* (2006.01)
*G16H 50/70* (2018.01)

(21) Application number: **20168300.0**

(22) Date of filing: **06.04.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **HIWALE, Sujitkumar Sureshrao
5656 AE Eindhoven (NL)**

• **JOHNSON, Mark
5656 AE Eindhoven (NL)**
• **RAMACHANDRAN, Ganesan
5656 AE Eindhoven (NL)**
• **CHAKRABARTI, Biswaroop
5656 AE Eindhoven (NL)**
• **BERA, Deep
5656 AE Eindhoven (NL)**

(74) Representative: **Philips Intellectual Property &
Standards
High Tech Campus 5
5656 AE Eindhoven (NL)**

(54) **APPARATUS AND METHOD FOR USE IN ASSESSING PLEURAL FLUID**

(57)      An apparatus (30) is for use in sensing and assessing pleural fluid in a subject. Embodiments comprise one or more sound sensors (40) for picking up sound signals from inside the chest subject, in particular respiratory sounds. The apparatus is arranged to acquire or derive a set of sound signals each corresponding to sounds received from a different intercostal location across the chest of the subject. There is also included a movement sensing means (36) which is configured at least to sense movement of the chest wall in and out with expiration and inspiration. A controller (32) is configured to process sound signals, in combination with the chest movement sensing data, for example in order to detect whether at each location the sound signals which are sensed are normal or abnormal with respect to typical pleural fluid status for the chest at that point. A comparison is made between characteristics of the sound signals for different locations, and also a correlation between the sound signals and the breathing cycle may be detected and these two factors used to assess each of the sound signals to assign it a classification as to whether the sound is normal or abnormal in relation to pleural fluid presence within the chest at that location.

FIG. 3

EP 3 893 247 A1

**Description**

FIELD OF THE INVENTION

The present invention relates to an apparatus and method for use in assessing pleural fluid, in particular based on analysis of sounds signals emitted from the chest.

BACKGROUND OF THE INVENTION

Pathologies of the respiratory system are one of the most common reasons for visits to an outpatient department. The respiratory system consists of a respiratory tract and the lungs. The lungs are the primary respiratory organs in human beings and are located in the thoracic cavity.

The lungs are covered on all sides by a thin layer of tissue membrane known as visceral pleura. The inner layer of the thoracic cavity is lined by another membrane known as parietal pleura. A sac-like space between these two pleural membranes is known as the pleural cavity and is filled with pleural fluid. The pleural fluid acts as a lubricant to ease expansion and contraction of the lungs during inspiration and expiration, thereby reducing effort required during breathing.

Pleural fluid is produced by the pleural layers via ultrafiltration of plasma and is reabsorbed by the lymphatic system. The production and reabsorption process of pleural fluid is balanced in such a way that only a small amount of pleural fluid (usually < 10 ml) is present in the pleural cavity at any given time.

However, a number of pathological conditions are known to disturb this delicate balance of production and reabsorption, leading to collection of excess fluid in the pleural cavity; this condition is known as pleural effusion.

A small pleural effusion may be relatively unproblematic. However, a large pleural effusion can lead to impaired breathing. A pleural effusion is usually diagnosed based on medical history and physical examination, and is confirmed by a chest X-ray.

A physical examination for pleural effusion usually involves percussion over the chest wall and auscultation using a stethoscope. During these methods, one side of the chest is used as a reference for the other side, with the assumption made that any pathological condition will be present on one side only, while the other side will be normal. However, this method has limitations in the case of bilateral pleural effusion, where the plural cavities on both sides are filled with fluid. In such cases it is not possible to use one side as a reference for the other side.

In the case of a unilateral and, sufficiently large, pleural effusion, bedside diagnosis using percussion and auscultation can provide an indication of pleural effusion. However, for small to moderate pleural effusions, or for loculated effusion, such physical examinations can fail to detect the pleural effusion.

Percussion involves tapping on a body surface to determine the underlying structure based on listening to the characteristics of sounds generated responsive to the tapping. The clinician is trained to know what sounds are normally generated when there is no pathology, and so can compare the sounds that they hear to their knowledge of the normal sounds. They can also compare the sounds to those on the other side of the chest wall.

As this method is based on the auditory perception of a doctor, it is highly subjective and thus can be unreliable. Moreover, percussion provides only an indication of the underlying structure without any information on the specific origin of sounds, meaning that more subtle analysis of any underlying pathology is not possible. For example both a consolidation of the lungs and a pleura effusion produce a dull note upon percussion, and so it is not possible to distinguish which pathological condition is present based just on percussion.

Auscultation involves listening to the intensity and quality of the lung sounds at different locations across the chest wall. Respiratory sounds (lung sounds) are generated due to the movement of air through the respiratory system and are audible across the chest and back. Any pathology, which hampers transmission of the respiratory sounds from the lung parenchyma to the chest wall results in diminished or absent respiratory sounds.

Pleural effusion is one such pathological condition where lung sounds are either diminished or absent, due in this case to collection of excess fluid between the lungs the and chest wall.

The clinician's skill and experience play an important role in pleural effusion detection, since many pathologies other than pleural effusion can be associated with diminished or absent respiratory sounds. Therefore, detection of pleural effusion by auscultation again relies on the auditory perception of a clinician, and thus is open to subjectivity and therefore at least partially unreliable. Furthermore, often a clinician cannot make a specific diagnosis of the particular respiratory pathology based on auscultation alone.

Furthermore, small pleural effusions are typically not detectable using auscultation, which limits its use, and means that other methods have to be used in conjunction with auscultation to fully assess the condition.

Another limitation of currently known assessment methods is that pleural fluid gravitates to the most dependent part of the pleural cavity (i.e. the gravitationally lowest part), which is not easily accessible for physical examination (e.g. back of the patient when the patient is supine).

A further limitation is that percussion can be painful if performed by inexperienced doctor or preformed repeatedly. Additionally, auscultation and percussion examinations require a quiet environment, which is not always possible in a busy outpatient environment.

An improved means for use in sensing and assessing pleural fluid of the subject and associated pathologies would therefore be of general advantage in this field.

SUMMARY OF THE INVENTION

The invention is defined by the claims.

According to examples in accordance with an aspect of the invention, there is provided a medical sensing apparatus for use in sensing pleural fluid of a subject, the apparatus comprising:

> a sensor arrangement of one or more sound sensors for detecting sounds from inside the chest of the subject;
> a movement sensing means for detecting at least movement of a chest wall of the subject;
> a controller, adapted to:
>
> > track a phase of the subject's breathing cycle based on chest wall movement data from the movement sensing means;
> > obtain from the sensor arrangement a plurality of sound signals corresponding to sounds received at the sensor arrangement via different intercostal space locations across the chest;
> > determine a set of one or more signal characteristics of each of the sound signals; and
> > apply a classification procedure in which, based on a comparison of the signal characteristics of the different sound signals with one another, and based on a temporal relationship of each sound signal with respect to the breathing phase over time, a classification for each sound signal is derived.

The classification may be related to or based on fluid presence in the chest at the corresponding chest location.

The classification may be based on whether the detected sounds at the given location are normal or abnormal, for example with regards to sounds expected for normal fluid presence or status at the location. This may be by reference to some prior information source, for example a reference dataset, or by use of a machine learning engine for example.

By acquiring sound signals from a plurality of different chest locations, and classifying each sound signal with reference to the signal characteristics of the sounds from other of the chest locations, essentially relative levels of the different sounds, relative to one another, can be established. In other words, there may be derived a relative distribution of the sound signal characteristics among the sound signals. This information is valuable in assessing fluid presence, since in different patients the particular sounds associated with fluid presence beneath the chest at a particular location may differ. By using a comparison of each signal with various other of the signals from other chest locations, signals which sound abnormal with respect to fluid presence or fluid status can be more easily or reliably established.

Furthermore, embodiments of the present invention also make use of chest movement data to track a phase of the breathing cycle. The nature of pleural fluid is that it may naturally move, slosh, or redistribute as the chest moves in and out with breathing, which can affect the sounds that are heard. Thus, pleural fluid presence is associated with characteristic variations in the detectable sound signals over the course of the breathing cycle. This information is thus highly valuable in assessing fluid presence or absence, or fluid status at different locations across the chest.

When this is combined with the comparison of detected sounds between different locations on chest, a very accurate and reliable assessment of presence or absence, or status of, pleural fluid within the chest beneath each corresponding location can be established.

The classification of each chest location's sounds may be based on a value of the one or more signal characteristics of each sound signal relative to an expected or normal value for a signal from the corresponding body location.

The classification can be performed based on use of some prior information source or ground truth, e.g. a reference dataset, or using a machine learning algorithm in which prior knowledge of normal or abnormal sound signal characteristic levels is implicitly embedded based on training of the algorithm.

In some examples, the controller may determine relative values of the signal characteristics of the different sound signals with respect to each other.

'Received via' means that the sounds captured in each sound signal are sounds which have passed through or have been transmitted through different intercostal space locations.

There are three main ways of acquiring the signals: one is to position a separate sound sensor at each different location on the chest, aligned with a different intercostal space location. Another is to use a single sound sensor or patch of sound sensors, for example integrated in a hand-held probe, and move it sequentially between different intercostal space locations to build up a set of sound signals. The third is to use an array of sound sensors, for instance integrated in a patch or other support structure, which can be mounted on the chest, and use acoustic steering or beamforming techniques to generate from incoming sound signals a plurality of output sound signals which correspond to sounds originating via different intercostal space locations across the chest.

The controller may be further configured to determine a status of the pleural fluid of the subject, e.g. normal pleural fluid level or abnormal pleural fluid level, and/or one or more properties or characteristics of pleural fluid such as a viscosity, volume, and/or density.

The classification is based at least in part on an intensity level of each sound signal. For example, the classification may be based on whether the intensity level is normal or abnormal for the given location. This may be based on reference to some prior information source, for example a reference dataset, or based on use of a machine learning algorithm, wherein the prior information is embedded in the training of the algorithm. For example, the classification may be based on whether the intensity level is above, below, or at a typical or average level for the given location, or within a defined normal range of levels.

The classification may be based on whether the intensity of the sound signals are enhanced or diminished compared to a normal level for the given location, for instance as recorded a reference dataset or other reference information source.

In accordance with one or more embodiments, the controller may be configured to determine a fluid level of the pleural fluid based on the classifications for different intercostal space locations.

This may be based on the spatial distribution of the classifications of the different sound signals across the chest. For example, diminished sounds can indicate presence of abnormal pleural fluid beneath the chest, whereas normal sounds indicate absence of excess pleural fluid. Thus, the vertical point of the chest (vertical being aligned with the height axis of the patient) where abnormal sound signals stop and normal sound signals begin might indicate a fluid level of the pleural fluid.

In one or more embodiments, the controller may be further configured to perform a comparison of the classifications for sound signals corresponding to matching intercostal space locations on different sides of the patient's chest. In the case of pleural pathologies, it can be valuable to compare the respiratory sounds from two different sides of the chest to determine any difference in sound properties. Differences can be indicative of a unilateral pleural pathology on one side. Thus by comparing the sound signals or the classifications for sounds at matching intercostal space locations but on opposite sides of the chest, a like-for-like comparison can be made between the two sides in a number of locations.

As discussed above, there are different options for the arrangement of sound sensors.

In some examples, the sensor arrangement may comprise an array of sounds sensors, for example with rows and columns.

In accordance with one or more embodiments, the controller may be configured to perform an initialization procedure comprising:

> processing sound signals received in use at the array of sound sensors at a plurality of different receiving locations across the chest of the subject, and to classify each of the different receiving locations as either rib-aligned or intercostal space-aligned, based on an intensity of sound signals received at each of the locations, and
> deactivating sound sensors located at the non-intercostal space locations, and keeping activated the sound sensors located at the intercostal-space locations.

This embodiment is particularly advantageous in cases where a single array of sound sensors is used, for example integrated in a patch for attachment to the chest or back. In this set of embodiments, the initialization procedure is for detecting the locations of ribs of the subject, in order to identify intercostal locations. The particular sound sensors of the array which are aligned with the intercostal locations may then be activated for use during the sensing procedure for assessing the pleural fluid, while those not aligned with intercostal spaces (i.e. aligned with ribs) can be deactivated. This provides a simple means for acquiring the plurality of sound signals corresponding to sounds received by different intercostal space locations, in this case using a single array of sound sensors.

In accordance with one or more embodiments, the system may comprise a patch integrating the plurality of acoustic sensors, the patch having a skin contact or skin adhering area, and the sensors arranged to be acoustically coupled with the skin via the skin contact area when the patch is worn.

In accordance with any embodiment of the present invention, the signal characteristics determined by the controller may include any one or more of: sound intensity, frequency, and spectral composition.

In accordance with one or more embodiments, in advance of determining the signal characteristics, and based on the tracked breathing phase, the controller may be configured to isolate sound signal components of the sound signals associated with breathing, and suppress or filter signal components not associated with breathing (for example associated instead with the heart).

This may for example be based on correlating the sound signals with the breathing phase signal. It may be based on detection of a frequency of the breathing, and applying a filter to the sound signals and to pass only those signal components which are within a certain range of the breathing frequency.

In accordance with one or more embodiments, the movement sensing means may be further configured to detect a posture of the subject. This may include detecting an orientation of the torso of the patient, for example detecting a tilt axis of the source of the patient relative to an upright position.

The controller may be configured to obtain a respective plurality of sound signals at different points during a movement of the patient from a first position to a second position.

As the patient moves between different positions, the fluid moves about within the pleural cavity, redistributing to the gravitationally lowest region. This movement of the fluid leads to characteristic changes in the detectable sound signals as it moves, enabling more reliable detection of the fluid. Furthermore monitoring sound signals as the fluid moves allows fluid properties of the signal to be established, for example properties related to viscosity, density, and/or volume of the fluid.

By way of example, the controller may be configured to compare the signal characteristics for the pluralities of signals at the different time points. As mentioned, this can be used to determine a status or condition of the pleural fluid, as well as one or more properties of pleural fluid, as well as potentially a status or condition of the patient, for example related to the pleural fluid status.

The points or times at which signals are acquired may include a time before the patient moves from the first position and a time after the patient has arrived at the second position.

The signal acquisition points may additionally include one or more points during the process of the movement (i.e. between the first and second position).

In accordance with one or more embodiments, the controller may be configured to determine one or more fluid properties of the pleural fluid based on changes in the sound signal characteristics at different points during the movement. The fluid properties may include for example viscosity, density and/or volume of the fluid.

This may be based on detecting and assessing a correlation between the tracked movement of the patient over time, and changes in the sound signal characteristics over time.

The controller may be further configured to determine a fluid type of the pleural fluid based on the determined fluid properties, for example whether the fluid is transudates or exudates fluid.

The controller may be configured to determine a sloshing frequency, and/or a Reynolds number associated with the fluid in some cases.

Examples in accordance with a further aspect of the invention also provide a method for assessing pleural fluid of a subject, comprising:

monitoring movement of chest wall of the subject to thereby track a phase of the subject's breathing cycle;
obtaining a plurality of sound signals corresponding to sounds received from inside the subject's chest via different intercostal space locations across the chest;
determining a set of one or more signal characteristics of each of the sound signals; and
applying a classification procedure comprising, based on a comparison of the signal characteristics of the different sound signals with one another, and based on a temporal relationship of each sound signal with respect to the breathing phase over time, deriving a classification for each sound signal.

The classification may be related to fluid presence at the corresponding location for the sound signal. It may be indicative of or related to normal or abnormal fluid presence.

The classification may be related to whether the sounds are normal or abnormal for that location in relation to fluid presence at said location.

In accordance with one or more sets embodiments, the method may comprise acquiring a first plurality of signals with the patient in a first position, and acquiring a second plurality of signals with the patient in a second position, and comparing signal characteristics of the two sets of signals to derive an assessment of the pleural fluid.

In one particular set of examples, in the first position, the patient may be lying in a lateral position, lying on a first side of the chest, and in the second position, the patient may be lying in a lateral positon on a second (opposite) side of the chest. This procedure is particularly advantageous for detecting small amounts of pleural fluid in the pleural cavity, which can be difficult to detect with the patient fully static. By moving the patient between these two extremal positions with the patient lying on different sides of their body, the movement of the fluid is detectable in changes in the characteristics of the sound signals between the two positions.

Examples in accordance with a further aspect of the invention comprise a computer program product comprising code means configured, when executed on a suitable computing device, to cause competing device to perform a method in accordance with any example or embodiment outlined above or described below.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically shows a cross-section through the chest of a subject, illustrating the pleural cavity;
Fig. 2 shows locations of an example set of intercostal spaces;
Fig. 3 shows in block diagram form an example apparatus in accordance with one or more embodiments;
Fig. 4 illustrates an example arrangement of a sound sensor arrangement suitable for use in accordance with one or more embodiments;
Fig. 5 outlines steps of an example workflow in accordance with one or more embodiments;
Fig. 6 outlines steps of a further example workflow in accordance with one or more embodiments;
Fig. 7 shows a graph illustrating relationship between pressure and perturbation frequency for a set of different liquids, for use in determining a fluid type of the pleural fluid;
Fig. 8 shows the relationship between Reynolds number and pressure for a fluid at different example perturbation frequencies; and
Fig. 9 illustrates the shape, and a set of example dimensions for, an example model of pleural cavity.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an apparatus for use in sensing and assessing pleural fluid in a subject. Embodiments comprise one or more sound sensors for picking up sound signals from inside the chest subject, in particular respiratory sounds. The apparatus is arranged to acquire or derive a set of sound signals each corresponding to sounds received from a different intercostal location across the chest subject. There is also included a movement sensing means which is configured at least to sense movement of the chest wall in and out with expiration and inspiration. A controller is configured to process sound signals, in combination with the chest movement sensing data in order to detect whether at each location the sound signals which are sensed are normal or abnormal with respect to typical pleural fluid status for the chest at that point. A comparison is made between characteristics of the sound signals for different locations, and also a correlation between the sound signals and the breathing cycle may be detected and these two factors used to assess each of the sound signals to assign it a classification as to whether the sound is normal or abnormal in relation to pleural fluid presence within the chest at that location.

Embodiments of the invention involve acquiring sound signals received at the sound sensors via different intercostal spaces across the chest. Intercostal spaces are gaps between the ribs.

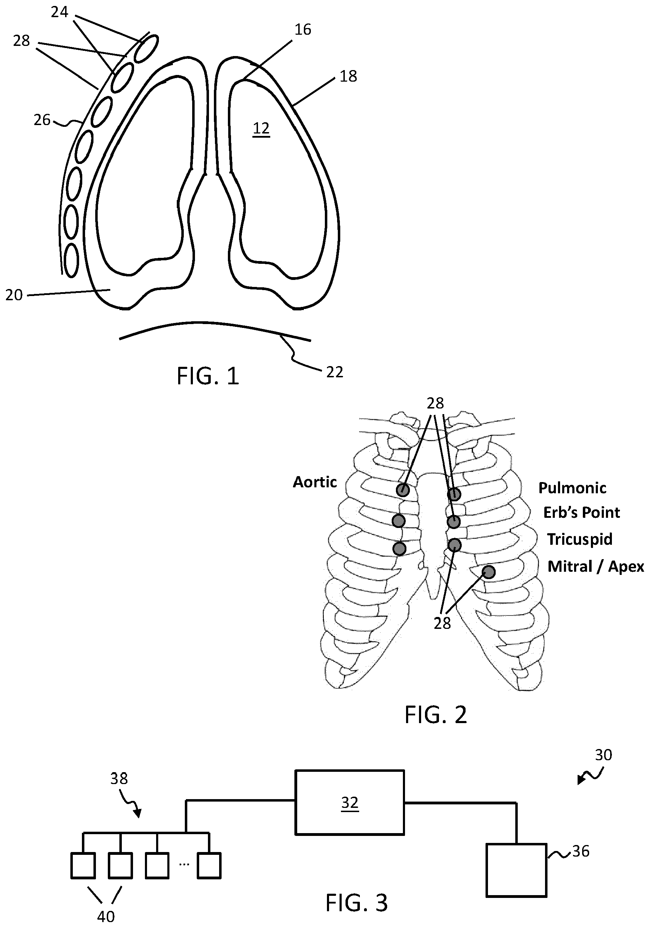

Fig. 1 for example schematically illustrates a cross-section of the chest of the subject. The figure shows the lungs 12. The lungs are covered on all external surfaces by a thin layer of tissue membrane known as the visceral pleura 16. The inner layer of the thoracic cavity is lined by another membrane known as the parietal pleura 18. A saclike space between these two pleural membranes is known as the pleural cavity 20 and it is this space that is filled with pleural fluid which acts as a lubricant. Beneath the lungs is shown the diaphragm 22. Also shown in fig. 1 is a cross-section of the ribs 24 of the subject, covered by the chest wall 26. The spaces between the ribs are known as intercostal spaces 28.

A further view of the ribs is schematically illustrated in Fig. 2. Fig. 2 shows one example selection of intercostal space locations 28 at which sound signals are typically acquired during an auscultation procedure. These locations include: the mitral valve, the tricuspid valve, aortic valve, and the pulmonary artery. These locations are just one nonlimiting selection of intercostal space locations. In particular, the illustrated locations are those typically used for auscultation of heart sounds. Respiratory sounds may be auscultated over larger areas including over trachea, chest, back and axillary areas. In certain embodiments of the invention, sound signals may be acquired from every intercostal space location, preferably on both sides chest, and optionally multiple signals may be acquired from a given intercostal space location. Thus, those illustrated in Fig. 2 are not limiting to the invention.

Fig. 3 schematically depicts an example apparatus in accordance with one or more embodiments, shown in block diagram form.

The apparatus 30 comprises a sensor arrangement 38 of one or more sound sensors 40 for detecting sounds from inside the chest of the subject.

The apparatus 30 further comprises a movement sensing means 36 for detecting at least movement of a chest wall of the subject. The movement sensing means may comprise one or more accelerometers for example. The one or more accelerometers may be for mounting to, or attachment to, the chest during use for example.

The apparatus 30 further includes a controller 32.

The controller 32 is configured to track a phase of the subject's breathing cycle based on chest wall movement data from the movement sensing means 36.

The controller 32 is further configured to obtain or acquire from the sensor arrangement 38 a plurality of sound signals corresponding to sounds received at the sensor arrangement 38 via different intercostal space locations 28 across the chest 26.

The controller 32 is configured to determine a set of one or more signal characteristics of each of the sound signals (for each of the different intercostal space locations 28 across the chest 26).

The controller 32 is further configured to apply a classification procedure. The classification procedure classifies the sounds detected at each of the intercostal space locations in relation to estimated fluid presence or fluid status in the chest at the corresponding location based on the sound signals from that location. The classification of each sound signal is based on a comparison of the signal characteristics of the different sound signals with one another, and based on a temporal relationship of each sound signal with respect to the breathing phase over time.

A classification for each sound signal is derived related to fluid status in the chest at the corresponding chest location. For example, each classification may be related to or indicative of whether the sounds detected at that location are normal or abnormal for that location with respect to normal (non-pathological) pleural fluid presence or status.

In some examples, the controller may be configured, by means of the comparison step to determine relative values of the signal characteristics of the different sound signals with respect to each other. In other words, there may be derived a relative distribution of the sound signal characteristics among the sound signals.

The sound sensors 40 are preferably for detecting just sub-ultrasonic sound signals. The sounds sensors may be or comprise microphones for example. However, other sound sensitive acoustic sensors may be used in other examples.

According to some examples, the apparatus may further comprise a memory unit communicatively coupled to the controller 32. The memory unit may have stored on it a reference dataset or database of reference 'normal' sound signals for different locations across the chest. In some embodiments, the classification procedure may comprise comparing the acquired sound signals with the reference dataset or database stored in this memory unit in order to determine whether each sound signal is normal or abnormal with respect to pleural fluid presence. This represents just one example means of implementing the classification process.

There may also be stored on the memory unit one or more algorithms for use in performing the classification procedure. These may be machine learning algorithms in some examples or they may be standard or classical computational algorithms.

There are different options for the sensor arrangement 38.

In one set of embodiments, the sensor arrangement comprises a plurality of sound sensors 40. The sensor arrangement may for example comprise an array of sound sensors. The array of sound sensors may be integrated in a support structure for attachment to or mounting to the chest or back. The support structure may for example take the form of a patch having a skin contact area by which the patch can be removably adhered to the subject. However, other support structures can also be used, such as one or more wearable units such as a chest strap or band structure or any other support structure.

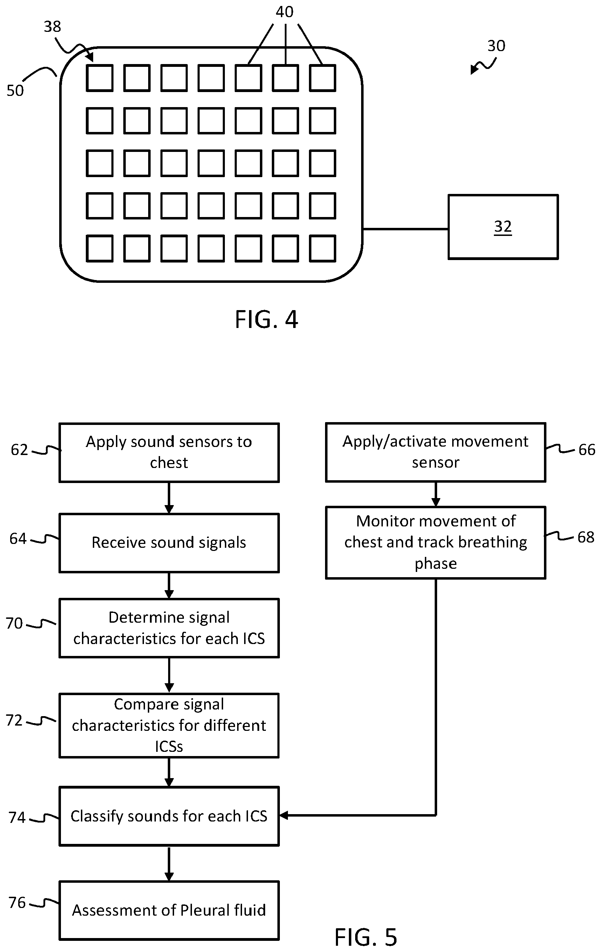

One possible example configuration for the apparatus is illustrated schematically in Fig. 4. In this example, the apparatus 30 comprises a skin adhesive patch 50. The skin adhesive patch has a skin contact area on one side by which the patch is attached to the chest or back. The arrangement 38 of sound sensors 40 is arranged in the form of an array, and the array 38 of sound sensors is integrated in the patch, with the sensors arranged to be acoustically coupled with the skin via the skin contact area of the patch when the patch is worn.

The patch may be applied to one side of the chest or can be placed such that it covers both the sides of the chest wall. Alternatively, according to some embodiments, two separate patches can be placed on two sides of the chest wall to cover identical areas. The sensors 40 of the two patches in this case may be operatively linked to a common controller so that the sound signals from both sides of the chest can be processed in assessing the pleural fluid.

The movement sensing means may be integrated also in the patch 50. The movement sensing means may comprise one or a plurality of movement sensors such as accelerometer sensors. Alternatively, the movement sensing means may be provided separate to the patch 50 or other support structure, for example as a separate sensor unit or element.

The use of a patch or other support structure integrating a plurality of sound sensors is just one example configuration.

According to a further set of examples, the apparatus 30 may comprise a plurality of acoustic sensors 40 not integrated in a support structure. They may be provided as separate, individual sensor units, each operatively coupled to the controller, e.g. via a wired or wireless connection. These may be attached in use to the subject's chest or back at appropriate locations, for example at each of the set of different intercostal locations across the chest for acquiring the plurality of sound signals from the different intercostal locations.

In a further alternative set of examples, the apparatus may be provided having just a single sound sensor, or a small set of sound sensors, and wherein the sensor arrangement is manually moved in use to different positions on the subject's chest to (sequentially) acquire sound signals at the different receive locations across the chest. This set of signals corresponding to the different receive locations is then received at the controller 32 which processes the signals in conjunction with the movement data to perform the classification of the different sounds.

As mentioned, the movement sensing means may comprise one or more accelerometers for attachment to the patient's body. In other examples however the movement sensing means may comprise a different form of movement sensor. For example, a gyroscopic position sensor could be used. In other examples, a noncontact movement sensor may be used such as an optical movement detector or a movement sensor which uses a different kind of electromagnetic emission. A camera might be used in some examples to track movement of the subject.

In use, the arrangement of sound sensors 40, e.g. the patch 50 integrating the sensors, is placed over the chest and/or back of the patient. The sound sensors are used to listen to sounds from the chest wall.

The movement sensor is used to detect at least the chest wall movement. The chest wall moves outward during inspiration and inwards during expiration. Based on the chest wall movements, respiratory phase of the respiratory cycle can be determined and tracked.

Sounds may be recorded from the sound sensors over a plurality of respiratory cycles.

The controller 32 receives a signal from both the sound sensors and the movement sensing means. The controller 32 may synchronize these two signals in some examples.

The movement sensing means 36 may continuously monitor chest movements during the sound recording.

There are two main types of pleural fluid assessment procedure which are compatible with embodiments of the invention: a first in which pleural fluid is sensed and assessed with the patient static, and a second is where pleural fluid is sensed as the patient moves between different positions. The second type of assessment can derive additional information about pleural fluid such as fluid properties. In one set of embodiments, the controller may be configured just for performing static assessments. In a further set of embodiments, the controller may be operable in different modes to perform either static assessments or assessments involving movement, or both.

In accordance with either set of embodiments, the static assessment procedure will now be discussed. Fig. 5 outlines in block diagram form an example set of steps which may be implemented by the controller in accordance with the set of embodiments.

As discussed, the arrangement of sound sensors is applied 62 to the chest or back ready for sensing sound signals. If the arrangement is integrated in a patch or other support structure, this may simply comprise attachment of the patch to the body. If the arrangement of sound sensors is a plurality of separate sound sensors, this may comprise attachment of the multiple sound sensors to appropriate locations.

The controller 32 receives 64 sound signals sensed by the arrangement 38 of sound sensors. In particular, the controller obtains from the sound sensors a plurality of sound signals corresponding to sounds received via different intercostal space locations across the chest.

As will be discussed in more detail below, this can be achieved in different ways. Separate sound sensors may be attached at each of a plurality of different intercostal space locations across the chest, with each sound sensor picking up sounds at a different location. In this way, the sound signals are generated by separate individual sound sensors. In other examples, in particular where an array, e.g. a patch, of sound sensors is used, a initialization or calibration procedure may be implemented to identify sound sensors which are aligned with particular intercostal space locations, and the controller may control only these sound sensors to supply sound signals to it for further processing, or may filter out sound signals from sound sensors not aligned with relevant intercostal space locations. In further examples, a form of acoustic beam steering or receive beamforming may be used to directionally focus sound pickup at only relevant intercostal space locations.

At the same time as the sound sensors are activated to supply sound signals, the movement sensing means 36 is activated 66 for tracking the movement of the chest to track 68 a breathing cycle of the subject.

The controller 32 determines 70 signal characteristics for the sounds received at each intercostal space location. The signal characteristics preferably include at least an intensity of the sound, and may also include spectral characteristics of the sound signal such as a central frequency or frequency spectrum for the sound signals. Any other signal characteristics may also be derived and used in the assessment.

The controller 32 is further configured to compare 72 the sounds recorded from the different intercostal space locations with one another. In particular, the signal characteristics for different sound signals are compared with one another. This may enable relative levels of the sounds of the different locations to be determined, i.e. relative to those of the other locations. In some cases, normalized or standardized sound signals for each location can be determined.

The controller is also configured to determine a relationship between each sound signal and the respiratory cycle. In particular a temporal relationship between the sound signal and the respiratory cycle may be determined. For example, the correlation between each sound signal and the respiratory cycle signal over time may be determined or assessed.

The comparison of each sound signal to the other sound signals and the relationship of each sound signal to the respiratory cycle is used in combination to classify 74 each sound signal for each of the different chest locations as discussed above. In addition, each sound signal may be compared with one or more reference sounds stored in a memory unit for performing the classification. The reference sounds may be normal sounds for each chest location. Thus by comparing the sound signals to the reference sounds, it can be established whether each sound signal for each location is normal or abnormal for that location, or a degree to which it is abnormal.

By way of example, based on intensity, spectral characteristics and relationship with the respiratory cycle, the recorded sounds for each location will be categorized as mentioned above. The categorization may be based on relative intensity and/or spectral characteristics (relative to the other sound signals) in some examples.

With regards the relationship with the respiratory cycle, the lung sounds vary cyclically (in amplitude and frequency) between inspiratory and expiratory phases of the respiration. This variation, if not taken into account, may distort the asessment of the sounds with respect to pleural fluid. Therefore, to differentiate between the natural variation due to the respiration and variation caused by the pleural fluid accumulation at specific locations it is advantageous to establish the relationship between the respiratory cycle and the recorded sounds and to use this as part of the categorisation.

By way of one possible example, the sound signal for each chest location might be categorized into one of four broad categories-(1) normal respiratory sounds; (2) accentuated (e.g. hyper resonant); (3) diminished; and (4) absent.

The classification of the sounds received via the different intercostal spaces may be done in different ways. The classification can be done using a single method or by combining multiple methods.

Classification of the recorded sounds may be a based on a predefined or dynamic threshold for intensity of the sound signals. For example, each chest location threshold may be set at a level above which sound is considered accentuated, and below which sound is considered diminished, and wherein accentuated and diminished sounds are indicative of a feature of fluid presence: either excess fluid, absence of fluid, or a boundary between fluid and no fluid in some cases.

According to one approach, a memory unit may be included in the apparatus which consists of a reference dataset of normal and abnormal respiratory sounds and algorithms to classify heart and lung sounds, and algorithms to classify different types of lung sounds. The reference dataset may comprise for each intercostal space location a reference set of normal or standard or expected signal characteristics (or ranges of such signal characteristics) for sounds originating from that location in the chest.

According to a further example, one or more machine learning algorithms may be used. The machine learning algorithms may be trained in advance with labelled training data comprising normal and/or abnormal sounds for different locations of the body and the algorithm trained to distinguish between the two for each location.

Techniques for classifying lung sounds at different chest locations as normal or abnormal are known in the art. The reader is referred for example to the paper: R. X. A. Pramono, S. Bowyer, and E. Rodriguez-Villegas, "Automatic adventitious respiratory sound analysis: A systematic review," PLoS ONE, vol. 12, no. 5, p. e0177926, 2017.

The paper R. Palaniappan, K. Sundaraj, and S. Sundaraj, "Artificial intelligence techniques used in respiratory sound analysis--a systematic review," Biomed Tech (Berl), vol. 59, no. 1, pp. 7-18, Feb. 2014 also contains details of suitable algorithms for determining the normal and abnormal lung sounds.

In accordance with one or more embodiments, the controller 32 may be configured to communicate the determined classifications for each of the sound signal locations to a user interface or other sensory output device, such as a display unit.

As mentioned, the controller performs a comparison of the signal characteristics of the different sound signals to each other. This may be for determining for each sound signal, a relative level or value of each of the signal characteristics relative to those of the other sound signals (the other locations in the chest). It may be therefore for essentially deriving a normalized or standardized level or value of each of the signal characteristics for each intercostal space location. It may be for deriving therefore an indication of a relative distribution of the signal characteristics across the different body signal locations.

In some examples, the reference values for each sound signal may be reference relative or normalized values for the signal characteristics in comparison with the other signals.

In some examples, the controller may be configured to perform a further step of deriving a status of the pleural fluid or performing assessment 76 of the pleural fluid. The controller may be configured for example to derive a parameter related to the pleural fluid. The controller may be configured to derive a fluid property of the pleural fluid.

In one set of examples, the controller may be configured to determine a fluid level of the pleural fluid in the chest, preferably a vertical fluid level of the pleural fluid up the chest, with the patient sitting upright.

Since in the present set of embodiments, the measurement is done with the patient static, and preferably sitting upright, any significant changes in patient posture or position (patient movements) during examination may introduce artefacts in the signals recorded by the microphones. Therefore in accordance with one or more embodiments, optionally, the accelerometer signal may be used to detect any significant changes in patient posture (patient movements). This information may then optionally be used to discard portions of any signals indicating substantial patient movements.

The sound sensors 40 will typically pick up both heart and respiratory sounds transmitted through the chest wall. Only the respiratory sounds are relevant for pleural fluid detection. To improve accuracy, in accordance with one or more embodiments, optionally, a filtering procedure may be applied to isolate or extract only signal components corresponding to lung activity.

In particular, in advance of determining the signal characteristics, and based on the tracked breathing phase, the controller may be configured to isolate sound signal components of the sound signals associated with breathing, and suppress or filter signal components not associated with breathing.

This may be done for example based on correlating the sound signals with the breathing phase signal, or for example based on detection of a frequency of the breathing cycle and then applying a filter to the sound signals to extract only the respiratory signal components.

By way of example, the received sounds may be classified by the controller 32 into two types (heart sounds or respiratory sounds) based on their frequency, intensity, spectral characteristics and/or relationship with the respiratory cycle. Only the respiratory sounds would be then used for further analysis. Example methods for classification of the heart and respiratory sounds are known in the art. The reader is referred for example to the paper: Gnitecki and Z. M. Moussavi, "Separating heart sounds from lung sounds," IEEE Engineering in medicine and biology magazine, vol. 26, no. 1, p. 20, 2007.

In accordance with one or more advantageous embodiments, the controller 32 may be configured to determine a fluid level of the pleural fluid in the chest. This may be done with the patient setup right, and a vertical fluid level up the chest of the patient determined.

The pleural cavity is a sac-like space and fluid in this cavity tends to gravitate to the most dependent part (the gravitationally lowest part) of the cavity. In an upright position, the small amount of fluid gravitates to the space between the posterior lower tip of the thoracic cavity and the diaphragm (the costophrenic recess). In such a condition, only the intercostal spaces, which are below the highest level of fluid will show diminished or absent respiratory sounds; whereas, the spaces above will have normal respiratory sounds. Thus, the controller can determine a level of the fluid based on a line or point up the vertical axis of the chest at which the sounds stop being diminished and start being normal.

In some cases, near the upper border of the fluid, the breath sounds may be accentuated due to increased conduction of breath sounds through the partially collapsed lung compressed by the fluid. Therefore, the microphones at the boundary may show accentuated breath sounds. In some cases, the controller may thus be configured to detect a spatial distribution of classifications which follows this pattern: diminished breath sounds where there is fluid, then accentuated breaths sounds at the fluid boundary and then normal breath sounds above the boundary. Detection of this pattern can be used to determine the fluid level of the pleural fluid.

In the case of pleural pathologies, it can be valuable to compare the respiratory sounds from the two sides of the chest or back to determine any differences in sound properties. These difference can be medically significant.

Thus, in one set of embodiments, the controller may be configured to further compare the signal characteristics of the respiratory sounds recorded from one side of the chest wall with the sounds recorded from the other side. The compared signal characteristics may include signal intensity and/or quality of the recorded sounds in some examples.

This comparison can be used to find out the side with diminished or absent respiratory sounds.

By way of example, an algorithm trained on a representative database of normal and abnormal sounds may be then used to classify whether sounds recorded from a particular pair of matching intercostal spaces is diminished or absent. An intercostal space (ICS) with diminished or absent respiratory sound may be then indicated to the user by audio-visual indicators.

The assessment of the respiratory sounds at rest is useful for detection of unilateral pathologies (e.g. unilateral pleural effusion). However, this approach alone cannot detect bilateral pleural effusion. Furthermore, even in relation to unilateral pathologies, there is only limited information that can be derived based on the sound signals of the fluid if it is static in a single position. For detecting or assessing features of some pathologies, it is necessary to acquire and compare sound signals with the fluid moved into different positions. In particular, in the case of seeking to detect pleural effusion, many conditions other than pleural effusion can lead to diminished or absent respiratory sounds in acquired sound signals. Thus it can be difficult to determine whether the underlying pathology is pleural effusion or a different pathology based just on the acquisition of sound signals with the patient in one position at rest.

In accordance with a further set of embodiments therefore, multiple sets of sound signals may be acquired from each of the different intercostal space locations with the patient moved into different positions, such that the pleural fluid is induced to move within the pleural cavity and the sound signals from different positions can be compared.

In accordance with this set of embodiments, the movement sensing means is preferably further configured to detect a posture of the subject. If the movement sensing means is an accelerometer, the same accelerometer can be used to detect patient posture as is used to detect chest wall movements. However, other sensing means for sensing patient posture might include optical position tracking means (for example a camera) or other contactless electromagnetic position tracking means, or a gyroscopic orientation detecting element. Various other patient posture detection means will be immediately apparent to the skilled person.

In accordance with this set of embodiments, the controller is configured to obtain a respective plurality of sound signals at different points during a movement of the patient from at least a first position to a second position. In particular, sound signals are acquired at least with the patient in the first position and in the second position. In other words, sound signals are acquired from the plurality of intercostal space locations before and after moving the patient between the positions.

For example, at least a first set of signals may be obtained with the patient in the first position, and then a second set of signals obtained with the patient moved to the second position.

The controller 38 may then be configured to compare the signal characteristics for the sets of signals acquired with the patient in the different positions.

In some embodiments, multiple sets of signals may be obtained during the process of movement of the patient so that movement of the fluid through the pleural cavity as a function of changing position of the patient can be detected and analyzed. This can be used to assess fluid properties of the pleural fluid for example.

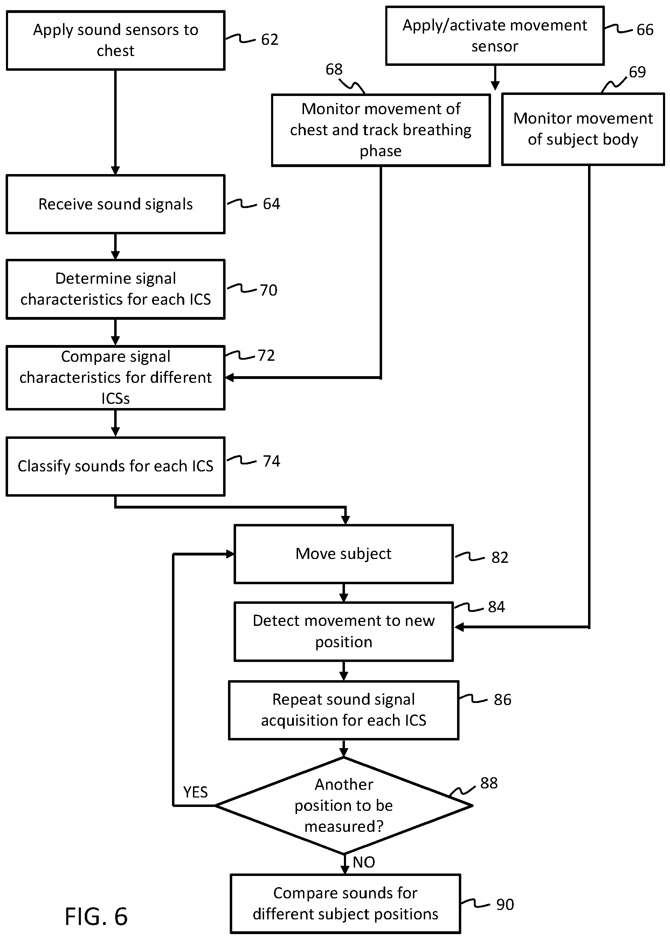

An example workflow according to one example embodiment in which sound signals are acquired with the patient in different positions is outlined in block diagram form in Fig. 6.

As in the static patient embodiment of Fig. 5, the arrangement of sound sensors is first applied 62 to the chest or back ready for sensing sound signals. At the same time, the movement sensing means 36 is applied and activated 66 for tracking the movement of the chest to track 68 a breathing cycle of the subject. In this case, the movement sensing means further includes means for monitoring posture of the patient. Thus the movement sensing means is also activated to monitor 69 movement of the subject's body, or monitor orientation or posture of the subject's body.

With the patient in a first position, for example sat or stood in an upright position, the same steps 64-74 discussed above in relation to the static patient embodiment of Fig. 5 are performed. Accordingly, the controller is configured to receive 64 sound signals corresponding to sounds from the plurality of ICS locations, determine 70 the signal characteristics for the sound signals of each ICS, compare 72 the signal characteristics for the different ICSs, and classify the sound signals for each ICS based on the comparison and based on the temporal relation between each of the sound signals and the respiration cycle of the patient, as tracked 68 by the movement sensing means.

The patient is then asked to move 82 their position to a second position. The second position for example has their chest at a different orientation, so that any pleural fluid shifts its distribution in the pleural cavity.

By way of example, the second position may have the patient with their chest tilted forward or backward, or more particularly tilted toward or away (or sideways) from the side of the chest where the arrangement of sensors is placed.

The patient may be asked to stay in the new (e.g. tilted position) for some time and then again move back to the neutral position.

The position sensor (e.g. accelerometer sensor) monitors 69 the movement of the subject and detects 82 when the patient is in the new position, and/or detects the orientation of the chest with the patient in the new position. In some examples, the movement sensing means may detect whether the patient moved toward or away from the patch. The position sensing means may also be used to derive angle of patient tilt.

The fluid will move in the pleural cavity with the patient's movements.

With the patient in the new, second position, steps 64-74 are repeated to acquire and process 86 the sound signals from each of the intercostal locations.

The sound signals may be acquired with the patient in multiple different positions, for example at different tilt angles of their torso chest, or with the chest tilted forward and then tilted backwards and/or tilted sideways in either direction. If sound signals at a further one or more patient positions are to be acquired, the controller is configured to repeat steps 82 to 86 for each new position.

Once the sound signals have been acquired with the patient in each of the positions, the respiratory sounds acquired with the patient in each of the different positions may be compared 90 to detect whether there is any change in the sound properties. For example, the signal characteristics of the sound signals from the different patient positions may be compared, and/or the classifications of the sounds for each intercostal space for the different patient positions may be compared.

For example, the sound signals from the different intercostal spaces will pick different intensity and quality of respiratory sounds dependent upon the position of the patient. For example, these properties may differ depending upon whether the patient has moved towards the microphone or away from it.

The comparison in the change in signal characteristics of the recorded sounds at the different patient positions may then be used to determine presence or absence of pleural fluid, or to determine one more properties or characteristics of the pleural fluid, or a status of the pleural fluid, or to detect one or more pathologies associated with the pleural fluid.

For example, in the absence of any lung pathologies, the respiratory sounds in two different positions of a patient would be almost the same, without any noticeable change in their properties. However, in case of pleural effusion for example, there would be change in sound properties depending on the direction of patient movement compared to the initial neutral position (the first position).

In an upright position, the fluid will settle to the most dependent part (gravitationally lowest) part of the pleural space. As the patient moves toward the side of their body in which the pleural effusion is present, the fluid will gravitate towards the tilted space and will block the transmission of the respiratory sounds from the chest wall in its vicinity. This will result in diminished or absent respiratory sounds in all the ICS below the fluid level. Whereas, when the patient moves away from pleural effusion side, the fluid will gravitate medially. As a result of this fluid shift, typically a subset of the intercostal spaces will become free of intervening pleural fluid, meaning that the sound signals detected via the spaces will change from exhibiting diminished sound signals (e.g. lower intensity than normal) to exhibiting normal sound signals (e.g. normal sound intensity).

Any change in sound properties between two positions of the patient may indicate that there is an underlying pathological condition.

An analysis of the change in sound properties at each ICS between different positions may be used in some examples to detect a fluid level of the pleural fluid in the pleural cavity. For example, an ICS number at which the pleural fluid level sits may be detected. The changes can also be used in some examples to detect whether one or both lungs have a pathological condition.

In preferred set of examples, the sound sensors are controlled to continuously or recurrently record sound signals from all the intercostal spaces during the course of the patient's movement from the first to the second position. The movement sensing means may also monitor the movement of the patient's body continuously or recurrently throughout the course of the patient's movement so that their position at any given time is detected.

In this way, the controller receives or obtains sound signals from the sound sensors at a plurality of time points during the course of the movement of the patient.

The patient position, as detected by the movement sensing means, may be temporally correlated with the acquired sound signals by the controller, so that the change in sound signal characteristics as a function of the patient position can be assessed.

For example, in a case of pleural effusion, sudden movements of a patient are likely to disturb the pleural fluid, resulting in turbulence, which could result in additional respiratory sounds. These are transient sounds without any relationship with the respiratory cycle. The intensity of these sounds is a function of the quantity of pleural fluid and magnitude of patient's movements. The signal from the movement sensing means can thus be used to detect when the patient is changing his or her position, and also detect the magnitude of the patient's movements. This information can then be correlated with the respiratory sounds recorded by the microphones to detect presence of additional sounds, which indicate presence of fluid in the pleural cavity.

In addition, certain fluid properties of the fluid can be detected based on correlating the movements of the patients, as detected by the patient movement sensing means, and the detected sound signal characteristics for the different ICSs at different points throughout the course of the patient's movement. The way in which the signal characteristics at each ICS varies as a function of patient movement can provide an indication for example about fluid viscosity, volume of fluid, fluid density. This will be explained in further detail further below.

In accordance with one or more embodiments, there may further be included a sensory output device, for example a user interface, by means of which the results of the pleural fluid assessment procedure can be communicated to a user. By way of example, the presence or absence of pleural effusion or a level of the pleural fluid in the pleural cavity (in relationship to the intercostal spaces) may be indicated to user. For example, the sensory output device could be a display unit, and/or one or more acoustic output devices. For example the output to the user could be an audio-visual output.

According to one or more embodiments, where a patch 50 is used integrating the plurality of sound sensors, a set of one or more light sources may be integrated in the patch, with their light output arranged to be visible to a user when the patch is affixed to a patient, i.e. arranged to emit light away from the direction of the skin contact area of the patch. Like sources of different colors may be included. The lights may be controlled to provide an indication of the underlying intercostal space or level of the pleural fluid within the chest. The lights could be also used to indicate change in the sound properties before and after movement for example.

In accordance with any of the above outlined embodiments, the steps performed by the controller include acquiring sound signals corresponding to a plurality of different intercostal locations across the chest.

As mentioned briefly above, this can be achieved in different ways depending upon the structural configuration of the arrangement 38 of sound sensors.

In accordance with one set of embodiments, the arrangement of sound sensors may be in the form of an array of sound sensors and provided integrated in a patch 50 for removable adherence to the chest or back of the subject (see Fig. 4, discussed above). The sensors are arranged in the patch such that, when the apparatus is in place on a subject's chest, each sensor is positioned at a different location across the chest. Each can therefore receive sound signals at a different location across the chest.

In accordance with this set of embodiments, the controller 32 may be configured to perform an initialization procedure to set up the sound sensors, in advance of acquiring and analyzing the sound signals for assessing the pleural fluid. This may be done as an initial step therefore.

The initialization procedure may be implemented to identify sound sensors which are aligned with particular intercostal space locations, and the controller may control only these sound sensors to supply sound signals to it for further processing, or may filter out sound signals from sound sensors not aligned with relevant intercostal space locations.

In particular, the initialization procedure may comprise applying or attaching the patch comprising the array of sound sensors to the chest, so that the sound sensors are then in position at a series of different receiving locations across the chest. Some of these will be aligned with intercostal spaces, and some aligned instead with locations of ribs which block propagation of sound.

The initialization procedure may then comprise processing sound signals received in use at the array 38 of sound sensors 40 at the plurality of different receiving locations across the chest of the subject, and to classify each of the different sensor locations as either rib-aligned or intercostal space-aligned, based on an intensity of sound signals received at each of the locations.

The controller may then be configured to deactivate sound sensors located at the non-intercostal space locations, and keep activated the sound sensors located at the intercostal-space locations.

In some examples, the arrangement 38 of sound sensors may comprise a plurality of subsets of sound sensors, each subset for sensing in use sound signals at a different receive location on the chest of the subject. The controller may be adapted to process the sound signals received at the arrangement of sound sensors, and to classify each of the subsets of sound sensors as either rib-aligned or intercostal space-aligned, based on an intensity of sound signals received at each of the subsets.

The arrangement may comprise an array having multiple rows, and wherein the subsets of the arrangement correspond to individual rows of the array.

Based on analysis of the intensities of the received sound signals at the array 38 of sound sensors 40, the controller 32 may estimate which sound sensors are positioned above ribs of the subject and which are above intercostal spaces.

Optionally, with this information, contours of the ribs may be determined and traced. A map of the (anterior) rib cage may optionally further be constructed. This may be a spatial map. The map may indicate positions of the ribs. It may include labels, e.g. numbers, for the ribs and/or intercostal spaces.

The controller may be adapted to classify a row as rib-aligned based on sensing a relative lower intensity sound signal, and to classify a row as intercostal space-aligned based on sensing a relative higher-intensity sound signal.

The rib bones intercept and attenuate sounds travelling from inside the body, leading to lower intensity pick-up at sensors positioned above ribs, than for sensors positioned above intercostal spaces.

Here, the controller is adapted to associate rib-alignment with a relative lower intensity sound signal, and is adapted to associate intercostal space alignment with a relative higher-intensity sound signal.

Hence, rows at which relative lower intensity signals are received are classified as rib-aligned, and rows at which relative higher intensity signals are received are classified as intercostal space-aligned.

In accordance with a further possible set of embodiments, instead of using an initialization process to keep activated only microphones aligned with intercostal locations, instead the controller may be configured to receive sound signals from all of the sound sensors of the array and to process them with a sound localization procedure. The localization procedure is for extracting sound signal components which correspond only to the locations of the intercostal spaces so that the signals used in the analysis of the pleural fluid are only those which have been received via these intercostal space locations.

The localization procedure may be for example an acoustic beam steering or acoustic receive beamforming procedure. It may involve for example applying a delay and sum procedure to the received acoustic signals from the plurality of sound sensors 40, this comprising applying an appropriate set of delays to the sound signals before summing them in order to emphasize signal components corresponding to the desired intercostal space locations.

An example sound signal localization procedure suitable for use in this set of embodiments is described for example in the paper: Tiete, J., Domínguez, F., da Silva, B., Segers, L., Steenhaut, K., & Touhafi, A. (2014). Sound-Compass: a distributed MEMS microphone array-based sensor for sound source localization. Sensors (Basel, Switzerland), 14(2), 1918-1949.

Use of a patch with an array of sound sensors is only one potential configuration.

In a further possible set of embodiments, a single sensor unit with a single sound sensor or set of sound sensors integrated therein may be used. This might for example be a handheld probe unit having the single sound sensor or set of sensors arranged acoustically coupled with a skin contact area of the probe unit.

The single sound sensor or set of sensors in the probe can be used to listen to sounds transmitted through patient's chest. The probe can be then used to get measurements from the different intercostal locations one at a time in a sequential manner. In some examples they may be acquired in a predefined sequence.

In particular examples, the controller may then be configured to process and compare the signals from the different locations to generate a chest surface map.

When the plural cavity contains only a very small amount of fluid, it is often difficult to detect it using conventional methods of physical examination.

According to one or more embodiments, the apparatus according to embodiments of the present invention can be used to detect presence of even a small amount of pleural fluid.

In particular, this can be done by acquiring sound signals at the plurality of intercostal space locations with the patient in each of at least two positions, wherein in the first position, the patient is lying in a lateral position, lying on a first side of the chest, and in the second position, the patient is lying in a lateral positon on a second side of the chest.

By way of example, in use, the patient may be asked to lie down in a lateral position with the arrangement of sound sensors attached to the chest for acquiring sound signals from a plurality of intercostal space locations. The sound sensors will be then used to detect respiratory sounds in accordance with steps 64 to 74 of the embodiment of Fig. 5 and Fig. 6 discussed above. The patient will be then asked change the position and to lie down in a lateral position on the opposite side of the chest. The steps 64 to 74 are then repeated for the patient in this new position. The signals recorded in these two positions will be then compared to detect any change in the properties of respiratory sounds.

In the case of pleural effusion, respiratory sounds will be diminished or absent in an ipsilateral position compared to when the patient is lying in the lateral position on the opposite side. This is different to what would be detected for other plural pathologies, where there would not be detected any change in the properties of the respiratory sounds in these two positions.

As discussed above, in accordance with one or more embodiments, the apparatus can be used to detect a level of pleural fluid within the chest. In other words, the apparatus can be used to quantify the level of fluid in the pleural cavity. One example implementation of this will now be discussed.

For this example, it is assumed that the apparatus comprises a patch 50 within which the arrangement of sound sensors is integrated. The patch is placed such that it covers the entire lateral surface of the chest from axilla to the lower border of the last palpable rib.

The respiratory sounds in the different ICS locations are recorded and classified in accordance with the steps 64 to 74 set out above in relation to Fig. 5. The sound signals acquired via all of the ICS locations below the level of the pleural fluid are likely to exhibit diminished or absent respiratory sounds.

Therefore, the first ICS along the direction of the chest from bottom of the chest toward the top (with the patient in an upright position) with normal or accentuated respiratory sounds will be considered to be aligned with an upper level of the pleura fluid. Whereas, the ICS just above the last rib will be considered to be aligned with the lowermost level of the pleural cavity.

The patch may be configured such that the array of sound sensors integrated therein have a uniform pitch, i.e. the spacing between neighboring sound sensors across the array may be uniform, so that all of the sound sensors in the patch are at a fixed distance from one another.

The length of the pleural fluid column may in this case be derived by calculating the distance between the sounds sensor aligned with the bottom-most ICS and the sound sensor aligned with the ICS detected as being coincident with an upper level of the pleural fluid.

As mentioned above, in accordance with one or more embodiments, the controller may be further configured to determine one or more fluid properties of the pleural fluid based on changes in the sound signal characteristics at different points during movement of the patient between one or more positions. The determined fluid properties may include for example fluid viscosity, density and/or volume.

The determination of the fluid properties may, in examples, be based on a correlation between the tracked movement of the patient over time, and changes in the sound signal characteristics over time.

In some examples, the controller may be configured to determine a fluid type of the pleural fluid based on the determined fluid properties, for example whether the fluid is transudates or exudates fluid. Exudate is inflammatory (it contains a plurality of additional components), whereas, transudate is a kind of filtered blood (watery) fluid.

An example approach for determining properties of the fluid, and/or type of the fluid will now be described below.

This approach is based on assessing a sloshing pressure (pressure exerted due to sloshing) and sloshing frequency of the pleural fluid. These properties have an effect on the detectable properties of sounds passing through the fluid.

The properties of a fluid that can affect the sloshing pressure (and consequent detectable sounds) are listed in Table 1 below. It is known that sloshing frequency and pressure are dependent on fluid properties such as (1) density, (2) viscosity and (3) volume. Moreover, the frequency of sloshing is also dependent on the acceleration applied to the fluid (the perturbation) and the characteristics (size and shape) of the container.

*Table 1*

| No. | Variable | Description | Dimension |
|---|---|---|---|
| 1 | a | Tank length | distance (L) |
| 2 | h | Undisturbed liquid height | L |
| 3 | 1 | Any length | L |
| 4 | ω | Disturbing frequency | 1/time (1/T) |
| 5 | t | Any time | T |
| 6 | β | Amplitude of disturbance | - |
| 7 | ρ | Density of liquid | $M/L^3$ |
| 8 | μ | Viscosity of liquid | M/LT |
| 9 | P | Any pressure | $M/LT^2$ |
| 10 | γ | Effective acceleration | $L/T^2$ |
| 11 | ζ | Surface tension | $M/T^2$ |

The viscosity and specific gravity (density) of transudate and exudate fluids are shown in Table 2 below.

*Table 2: Specific gravity and viscosity of transudate and exudate.*

| Parameter | Transudate | Exudate |
|---|---|---|
| Specific gravity | < 1.012 | > 1.020 |
| Viscosity | $0.9370 \pm .03$ mPa-s | $1.377 \pm 0.16$ mPa-s |

The effect of viscosity and density on sloshing pressure for a given volume of liquid has been investigated in the study described in the paper: Zou et al. "The effect of liquid viscosity on sloshing characteristics," Journal of Marine Science and Technology 20(4), 2015. DOI: 10.1007/s00773-015-0329-y).

The viscosity of four different liquids used in this study is shown in Table 3 below.

*Table 3: Density and viscosity of liquid used in the Zou et al study*

| Name | Density (kg/m$^3$) | Viscosity (N s m$^{-2}$) |
|---|---|---|
| Liquid_1 (water) | 998 | 0.00152 |
| Liquid_2 | 998 | 0.095 |
| Liquid_3 | 998 | 0.176 |
| Liquid_4 | 998 | 0.25 |

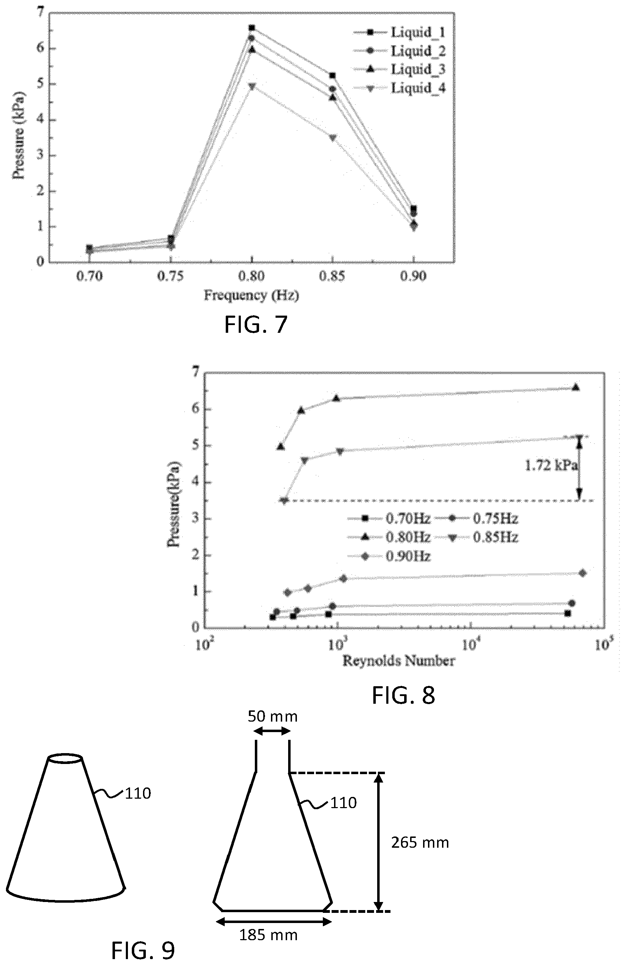

Fig. 7 shows the relationship between the perturbation frequency (x-axis) and sloshing pressure (y-axis) for the liquids listed in Table 3, having different viscosities.

Fig. 8 further shows a relationship between the sloshing pressure (y-axis) and Reynolds number (x-axis). This relationship was established in the study mentioned above from paper: Zou et al. "The effect of liquid viscosity on sloshing characteristics," Journal of Marine Science and Technology 20(4), 2015. DOI: 10.1007/s00773-015-0329-y.

The Reynolds number of a fluid can be determined using the equation:

$$Re = \frac{u_m d_0}{\upsilon} = \frac{\rho u_m d_0}{\mu} = \frac{2\rho \pi f \theta_{\text{roll}} r_{\max} d_0}{\mu}$$

.

where $u_m$ is the maximum velocity of the tank, $f$ and $\theta_{roll}$ are the excitation frequency and the rolling amplitude of the tank respectively, $r_{max}$ is the maximum radius of rotation of the tank for rolling motion, $v$ and $\mu$ are kinematic viscosity and dynamic viscosity of the liquid, respectively, $d_0$ is the liquid depth when still, and $\rho$ is the density of the liquid.

The Zou et al study indicates that due to the differences in both viscosity and specific gravity of different fluids, the sloshing sound produced by the two types of pleural fluids (transudate and exudate) will be very different.

Below are outlined steps of example procedure for determining the volume and type of the pleural fluid (transudates or exudates).

Step 1: Detect the frequency of vibration (perturbation frequency) of the fluid, based on assuming that the frequency of movement of the chest wall during the patient's movement will correspond to a frequency of perturbation of the pleural fluid. This frequency can be acquired using the movement sensing means 36 or using the sound sensors, or both.

Step 2: The empirical sloshing frequency is determined using the following formula (taken from the paper: Jaiswal et al., "A study on sloshing frequencies of fluid-tank system," The 14th World Conference on Earthquake Engineering, October 12-17, 2008, Beijing, China):

$$f_c = \frac{1}{2\pi}\sqrt{\frac{3.68g\,\tanh\left(3.68\,\dfrac{h}{D}\right)}{D}}$$

In this formula, fc = Sloshing frequency (Hz), h = height of water in tank (m), D= diameter of circular tank/cavity (m), g = acceleration due to gravity ($ms^{-2}$). These parameters are estimated based on modelling the pleural cavity as being approximately conical in shape. By way of one possible example, an approximate set of dimensions for the model conical pleural cavity are illustrated in Fig. 9. These represent one example set of dimensions only.

The left side of Fig. 9 illustrates the model conical shape 110 in perspective view, and the right side of Fig. 9 shows a cross-section through the conical shape for this model. The example set of approximate dimensions for the pleural cavity on this model are shown in Fig. 9. The above equation assumes a conical shaped pleural cavity.

Different values of the coefficients for the equation can of course be used in further examples, for example based on establishing a more accurate assumption about the shape of the pleural cavity and the various dimensions of it.

In the case of pleural effusion, *h* could be determined from a static measurement with the patient in an upright position, based on the procedure for detecting a level of pleural fluid in the chest outlined in detail above.

*D* may be approximately calculated based on an anatomical model known from the art, using the chest circumference, height and gender of the patient. Many different anatomical models and techniques for determining such parameters (such as chest wall and lung volume) are known in the field. By way of example, the reader is referred to the paper: S. J. Cala et al., "Chest wall and lung volume estimation by optical reflectance motion analysis," Journal of Applied Physiology, vol. 81, no. 6, pp. 2680-2689, Dec. 1996.

Step 3: Compute the approximate volume of the pleural cavity using D, assuming the shape of the model pleural cavity of Fig. 9, and the percentage of fluid filled volume (p%) (based on the determined fluid level in the cavity).

Step 4: The model showed in Fig. 9 does not consider the lungs, which are suspended in the pleural cavity. To calculate the effective volume available for pleural fluid, the lung volume calculated from the anatomical model in step 2 can be subtracted from pleural cavity volume calculated in step 3.

Step 5: Compute the Reynold's number (using equation mentioned above), and obtain the magnitude of pressure using the curves shown in Fig. 8, based on the frequency of sloshing obtained in Step 1.

Step 6: For a given *p* (%), if the amplitude of pressure is above a certain threshold, then the pleural fluid is transudates type or the pleural fluid is exudates type. These thresholds can be derived empirically or through modelling, based on deriving pressure versus frequency curves for different fluids of different types (at different *p* (%) values), such as those shown in Fig. 8.

For example, using the example curves in Fig. 7, for a pleural fluid for which: p = 20%, fc = 0.8 Hz, Re = 1000 and pressure is 6.5 kPa, then the fluid is transudates.

In examples discussed above, an example movement sensing means 36 which employs an accelerometer has been mentioned. However there are many possibilities for the movement sensing means.

For example, in accordance with one or more embodiments, movement sensing means may employ contactless movement sensing techniques, for example using electromagnetic emissions to track patient movement, for example using optical tracking of patient. For example one or more video cameras may be employed in accordance with some examples to determine patient's movement or orientation.

In other implementation, other methods can be used to determine patient's movement or orientation. For example, a motorized patient bed or chair might be employed, able to adjust an angle of the upper body of the patient, and to communicate the angle to the controller 32. Thus in this case, the motorized bed or chair would perform the role of the movement sensing means 36.

Examples in accordance with a further aspect the invention also provide a method for assessing pleural fluid of a subject, comprising:

monitoring 68 movement of chest wall of the subject to thereby track a phase of the subject's breathing cycle; obtaining 64 a plurality of sound signals corresponding to sounds received from inside the subject's chest via different intercostal space locations across the chest; determining 70 a set of one or more signal characteristics of each of the sound signals; and applying 74 a classification procedure comprising, based on a comparison 72 of the signal characteristics of the different sound signals with one another, and based on a temporal relationship of each sound signal with respect to the breathing phase over time, deriving a classification for each sound signal related to fluid presence at said location.

Implementation options and details for each of the above steps may be understood and interpreted in accordance with the explanations and descriptions provided above for the apparatus aspect of the present invention (i.e. the medical sensing apparatus).

Any of the examples, options or embodiment features or details described above in respect of the apparatus aspect of this invention (in respect of the medical sensing apparatus) may be applied or combined or incorporated mutatis mutandis into the present method aspect of the invention.

Examples in accordance with a further aspect of the invention provide a computer program product comprising code means configured, when executed in a processor, operatively coupled with a sensor arrangement of one or more sound sensors for detecting sound signals from inside the chest of the subject, and a movement sensing means for detecting movement of the chest wall of the subject, to cause the processor to perform the method in accordance with any example or embodiment outlined above or described below, or in accordance with any claim of this application.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A medical sensing apparatus (30) for use in sensing pleural fluid of a subject, the apparatus comprising:

a sensor arrangement (38) of one or more sound sensors (40) for detecting sounds from inside the chest of the subject;

a movement sensing means (36) for detecting at least movement of a chest wall of the subject;

a controller (32), adapted to:

track (68) a phase of the subject's breathing cycle based on chest wall movement data from the movement sensing means;

obtain (64) from the sensor arrangement a plurality of sound signals corresponding to sounds received at the sensor arrangement via different intercostal space locations across the chest;

determine (70) a set of one or more signal characteristics of each of the sound signals; and

apply (74) a classification procedure in which, based on a comparison (72) of the signal characteristics of the different sound signals with one another, and based on a temporal relationship of each sound signal with respect to the breathing phase over time, a classification for each sound signal is derived related to fluid presence in the chest at the corresponding chest location.

2. An apparatus as claimed in claims 1, wherein the classification is based at least in part on an intensity of each sound signal.

3. An apparatus as claimed in claim 1 or 2, wherein the controller is configured to determine a fluid level of the pleural fluid based on the classifications for different intercostal space locations.

4. An apparatus as claimed in any of claims 1 to 3, wherein the controller is further configured to perform a comparison of the classifications for sound signals received via matching intercostal space locations on opposite sides of the patient's chest.

5. An apparatus as claimed any of claims 1-4, wherein the system comprises a patch integrating the plurality of acoustic sensors, the patch having a skin contact area, and the sensors arranged to be acoustically coupled with the skin via the skin contact area when the patch is worn.

6. An apparatus as claimed in any of claims 1-5, wherein said signal characteristics include one or more of: intensity, frequency, and spectral composition.

7. An apparatus as claimed in any of claims 1-6, wherein, in advance of determining the signal characteristics, and based on the tracked breathing phase, the controller is configured to isolate sound signal components of the plurality of sound signals associated with breathing, and suppress or filter signal components not associated with breathing.

8. An apparatus as claimed in any of claims 1-7, wherein the movement sensing means is further configured to detect a posture of the subject, and wherein the controller is configured to obtain a respective plurality of sound signals at different points during a movement of the patient from a first posture position to a second posture position, and preferably wherein controller is configured to compare the signal characteristics for the pluralities of signals at the different acquisition points.

9. An apparatus as claimed in claim 8, wherein the points include a time before the patient moves from the first position and a time after the patient has arrived at the second position.

10. An apparatus as claimed in claim 9, wherein the points additionally include one or more points during the process of the movement.

11. An apparatus as claimed in any of claims 8-10, wherein the controller is configured to determine one or more fluid properties of the pleural fluid based on changes in the sound signal characteristics at different points during the movement, for example viscosity, density and/or volume of fluid.

12. An apparatus as claimed in claim 11, wherein the controller is further configured to determine a fluid type of the pleural fluid based on the determined fluid properties, for example whether the fluid is transudates or exudates fluid.

13. A method for assessing pleural fluid of a subject, comprising:

monitoring (68) movement of chest wall of the subject to thereby track a phase of the subject's breathing cycle;

obtaining (64) a plurality of sound signals corresponding to sounds received from inside the subject's chest via different intercostal space locations across the chest;
determining (70) a set of one or more signal characteristics of each of the sound signals; and
applying (74) a classification procedure comprising, based on a comparison (72) of the signal characteristics of the different sound signals with one another, and based on a temporal relationship of each sound signal with respect to the breathing phase over time, deriving a classification for each sound signal related to fluid presence at said location.

14. A method as claimed in claim 13, wherein the method comprises acquiring a first plurality of signals with the patient in a first position, and acquiring a second plurality of signals with the patient in a second position, and comparing signal characteristics of the two sets of signals to derive an assessment of the pleural fluid, and
optionally wherein in the first position, the patient is lying in a lateral position, lying on a first side of the chest, and in the second position, the patient is lying in a lateral positon on a second side of the chest.

FIG. 1

Aortic

Pulmonic

Erb's Point

Tricuspid

Mitral / Apex

FIG. 2

FIG. 3

FIG. 4

| | |
|---|---|
| 62 — Apply sound sensors to chest | Apply/activate movement sensor — 66 |
| 64 — Receive sound signals | Monitor movement of chest and track breathing phase — 68 |
| 70 — Determine signal characteristics for each ICS | |
| 72 — Compare signal characteristics for different ICSs | |
| 74 — Classify sounds for each ICS | |
| 76 — Assessment of Pleural fluid | |

FIG. 5

FIG. 6

FIG. 7

FIG. 8

FIG. 9

Europäisches
Patentamt

European
Patent Office

Office européen
des brevets

# EUROPEAN SEARCH REPORT

Application Number

EP 20 16 8300

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/119255 A1 (MAHAJAN AMAN [US] ET AL) 4 May 2017 (2017-05-04) * paragraphs [0012], [0017], [0035] - [0037], [0046], [0055], [0072]; figure 1 * | 1-14 | INV. G16H50/20 A61B5/08 A61B5/11 A61B5/113 G16H50/70 |
| A | US 2018/125444 A1 (KAHLMAN JOSEPHUS ARNOLDUS HENRICUS MARIA [NL] ET AL) 10 May 2018 (2018-05-10) * paragraph [0035] * | 1-14 | |
| A | Physiopedia: "Auscultation", , 6 August 2017 (2017-08-06), XP055719122, Retrieved from the Internet: URL:https://web.archive.org/web/2017080604 4213/https://physio-pedia.com/Auscultation [retrieved on 2020-07-30] * section "Respiratory Examination" * | 1-14 | |
| A | Physiopedia: "Lung Sounds", , 30 June 2017 (2017-06-30), XP055719123, Retrieved from the Internet: URL:https://web.archive.org/web/2017063021 1217/http://www.physio-pedia.com/Lung_Soun ds [retrieved on 2020-07-30] * section "Soft Breath Sounds" * | 1-14 | TECHNICAL FIELDS SEARCHED (IPC) G16H A61B |

-/--

The present search report has been drawn up for all claims

| Place of search | Date of completion of the search | Examiner |
|---|---|---|
| Munich | 31 August 2020 | Reinbold, Bernhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches Patentamt
European Patent Office
Office européen des brevets

# EUROPEAN SEARCH REPORT

Application Number

EP 20 16 8300

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GNITECKI J ET AL: "Separating Heart Sounds from Lung Sounds - Accurate Diagnosis of Respiratory Disease Depends on Understanding Noises", IEEE ENGINEERING IN MEDICINE AND BIOLOGY MAGAZINE, IEEE SERVICE CENTER, PISACATAWAY, NJ, US, vol. 26, no. 1, 1 January 2007 (2007-01-01), pages 20-29, XP011183790, ISSN: 0739-5175, DOI: 10.1109/MEMB.2007.289118 * the whole document * ----- | 1-14 | |

TECHNICAL FIELDS
SEARCHED      (IPC)

The present search report has been drawn up for all claims

| Place of search | Date of completion of the search | Examiner |
|---|---|---|
| Munich | 31 August 2020 | Reinbold, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**EP 3 893 247 A1**

## ANNEX TO THE EUROPEAN SEARCH REPORT
## ON EUROPEAN PATENT APPLICATION NO.

EP 20 16 8300

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2020

| Patent document cited in search report | Publication date | Patent family member(s) | Publication date |
|---|---|---|---|
| US 2017119255 A1 | 04-05-2017 | AU 2015259002 A1<br>CA 2947476 A1<br>CN 106456017 A<br>EP 3142546 A1<br>JP 6678118 B2<br>JP 2017521106 A<br>KR 20170007286 A<br>US 2017119255 A1<br>WO 2015175904 A1 | 10-11-2016<br>19-11-2015<br>22-02-2017<br>22-03-2017<br>08-04-2020<br>03-08-2017<br>18-01-2017<br>04-05-2017<br>19-11-2015 |
| US 2018125444 A1 | 10-05-2018 | CN 107529991 A<br>EP 3282950 A1<br>JP 2018516616 A<br>US 2018125444 A1<br>WO 2016166318 A1 | 02-01-2018<br>21-02-2018<br>28-06-2018<br>10-05-2018<br>20-10-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. X. A. PRAMONO ; S. BOWYER ; E. RODRIGU-EZ-VILLEGAS.** Automatic adventitious respiratory sound analysis: A systematic review. *PLoS ONE,* 2017, vol. 12 (5), e0177926
- **R. PALANIAPPAN ; K. SUNDARAJ ; S. SUNDA-RAJ.** Artificial intelligence techniques used in respiratory sound analysis--a systematic review. *Biomed Tech (Berl),* February 2014, vol. 59 (1), 7-18
- **GNITECKI ; Z. M. MOUSSAVI.** Separating heart sounds from lung sounds. *IEEE Engineering in medicine and biology magazine,* 2007, vol. 26 (1), 20
- **TIETE, J. ; DOMÍNGUEZ, F. ; DA SILVA, B. ; SEGERS, L. ; STEENHAUT, K. ; TOUHAFI, A.** Sound-Compass: a distributed MEMS microphone array-based sensor for sound source localization. *Sensors (Basel, Switzerland,* 2014, vol. 14 (2), 1918-1949
- **ZOU et al.** The effect of liquid viscosity on sloshing characteristics. *Journal of Marine Science and Technology,* 2015, vol. 20 (4
- **ZOU et al.** The effect of liquid viscosity on sloshing characteristics. *Journal of Marine Science and Technology,* vol. 20 (4), 2015
- **JAISWAL et al.** A study on sloshing frequencies of fluid-tank system. *The 14th World Conference on Earthquake Engineering,* 12 October 2008
- **S. J. CALA et al.** Chest wall and lung volume estimation by optical reflectance motion analysis. *Journal of Applied Physiology,* December 1996, vol. 81 (6), 2680-2689